# EUROPEAN PATENT APPLICATION

(11) **EP 3 944 820 A1**
(43) Date of publication of application: **02.02.2022**
(21) Application number: 20188110.9
(22) Date of filing: 28.07.2020
(51) Int. Cl.: A61B 8/08, A61B 17/34

(54) **INTERVENTIONAL DEVICE WITH AN ULTRASOUND TRANSCEIVER**

(71) Applicant: Koninklijke Philips N.V., 5656 AG Eindhoven (NL)
(72) Inventor: ERKAMP, Ramon Quido, 5656 AE Eindhoven (NL); CHEN, Alvin, 5656 AE Eindhoven (NL); BHARAT, Shyam, 5656 AE Eindhoven (NL); VAIDYA, Kunal, 5656 AE Eindhoven (NL); JAIN, Ameet Kumar, 5656 AE Eindhoven (NL); VIGNON, Francois Guy Gerard Marie, 5656 AE Eindhoven (NL)
(74) Representative: Philips Intellectual Property & Standards

(57) **Abstract**

A system and a method for determining a relative position of an ultrasound transceiver with respect to an opening of an interventional device where the opening is within a lumen of the interventional device. Ultrasound transmissions from an ultrasound transceiver, capable of moving through at last part of the lumen and the opening are reflected off the wall or boundary of the lumen. The ultrasound transceiver may receive one or more echoes of the ultrasound transmissions and generate a receive signal comprising the one or more echoes and transmit receive data comprising one or more characteristics of the echo to a data processor. The data processor is configured to identify from the receive data the one or more characteristics and determine a relative position of the ultrasound transceiver with respect to the opening based on the one or more characteristics. A transceiver may be part of a retrofit device with which the interventional device may be equipped during an interventional procedure. After having determined the relative position, a location within a subject of the interventional device equipped with the retrofit device may be determined through tracking of the transceiver by an US imaging apparatus during the procedure.

## Description

### FIELD OF THE INVENTION

The invention relates to the field of retrofitting an interventional device with a transceiver and with determining a relative position of the transceiver with respect to the interventional device.

### BACKGROUND OF THE INVENTION

During medical procedures, it is often desirable to know the location of an interventional device inserted into the body of a subject such as a patient. Interventional devices, such as for example a catheter or a needle of a syringe, are generally made of highly reflective materials with smooth surfaces providing low scattering in multiple directions; the visibility of these devices in ultrasound images is often therefore rather poor, as ultrasound imaging beams are reflected away from the ultrasound probe.

Various solutions have been proposed to alleviate this problem. Devices have been manufactured with echogenic coatings, but this provides only a limited improvement in visibility. Ultrasound images system manufacturers have developed algorithms that use multiple imaging beams from various angles; again, this offers a limited improvement, and, further, is suitable only for linear arrays. Neither of these solutions can be used to detect devices that are inserted perpendicularly, or almost perpendicularly, to the ultrasound imaging plane.

Another solution is to add an ultrasound transducer at or near a fixed position at the tip of the interventional device. Imaging beams transmitted by an ultrasound imaging probe outside the subject and received by such transducer, or imaging beams transmitted by such transducer and received by an ultrasound imaging probe outside the subject, may be used to determine the location of the tip of the device in a subject through determination of the location of the transducer within the subject.

This latter approach requires that an interventional device is permanently equipped with the transducer and existing interventional devices without such transducer may not benefit from the location determination. There is therefore a need for a mechanism that enables existing interventional devices to be retrofitted with a transducer such that it may serve to locate the device through location of the transducer.

### SUMMARY OF THE INVENTION

The invention is defined by the claims.

According to examples or embodiments in accordance with aspects of the current disclosure, there are provided a device comprising a data processor circuit and a system comprising such device.

The system, device and/or data processor circuit is able to receive or generate data that comprises at least part of a receive signal provided by an ultrasound transceiver where the receive signal is caused by ultrasound echoes of ultrasound transmitted by the ultrasound transceiver. The ultrasound transceiver is adapted to be movable within a lumen of an interventional device and with respect to an opening within the interventional device. The receive signal comprising one or more echoes may be used to determine a relative position of the transceiver with respect to the opening.

Thus, the system, device and/or data processor circuit is able to identify form the receive data one or more characteristics of at least one echo and determine therefrom the relative position of the ultrasound transceiver with respect to the opening of the interventional device.

The device may be a processor circuit, a computer such as for example mobile computer, laptop, mobile phone, tablet, or a non-mobile one such as desktop computer or workstation. It may be part of a console of an ultrasound device or system. Such system may be an Ultrasound imaging device or system.

The interventional device may be any device envisaged to be tracked within a subject that has the lumen or hollow portion with an opening, for example at a distal tip, to its outside environment. Non limiting examples comprise: an endoscope, catheter, needle of a syringe, surgical tool etc. The transceiver is capable of moving through the lumen towards or away from the opening and possibly and preferably even through the opening, but this is not strictly necessary. It may thus be slidably movable. A hollow catheter capable of receiving a guidewire inside its lumen is one example.

The ultrasound transceiver, which may also be referred to as a transducer, is capable of transmitting and receiving ultrasound. Ultrasonic transceivers in the context of this disclosure are transducers that can generate and sense ultrasound energy. A transceiver can thus comprise an ensemble of at least one transducer configured to transmit ultrasound and at least one separate transducer configured to receive ultrasound. Additionally, or alternatively it can comprise at least one transducer configured to transmit and receive ultrasound. Transmitters convert electrical signals into ultrasound, receivers convert ultrasound into electrical signals, and transceivers can both transmit and receive ultrasound. The ultrasound may be transmitted in the form of pulses, in order that a single transceiver may be used to both transmit and receive, and in order to easily determine the time delay between transmission and reception.

Ultrasound transmissions of the transceiver may be reflected by the interventional device such as for example by the inner wall or boundary of the interventional device, if it is within appropriate distance from the transceiver. The transceiver is further capable of receiving one or more ultrasound echoes caused by such reflections and providing receive signals comprising the one or more echoes. As described an indication of the relative position of the transceiver with respect to the opening may be determined.

The approach allows retrofitting an interventional device with an ultrasound transducer in order that the ensemble may be used of determining the location of the interventional device with ultrasound. For example, when the relative position has been determined and possibly frozen of fixed while the transceiver can communicate ultrasound with a further ultrasound imaging probe, then its location may be tracked using the imaging probe. Using the determined relative position, the so tracked location may be translated into the location of the interventional device or a part of it such as the opening. Hence the system device and processor circuit may be used to guide navigation of the interventional device to a desired location. Once a desired interventional device location has been reached, the transceiver may be removed to restore the original functionality of the interventional device. Thus, system, device or processor circuit may be used to guide the positioning of the tip of an interventional device such as for example a catheter or needle while it is inserted into a subject during a medical procedure.

There are several reasons why a clinician may wish to retrofit a interventional device with an ultrasound transducer and determine the position of this transducer with respect to the interventional device while the interventional device is inserted into a subject. For example, the insertion of the interventional device into the patient may result in an unknown shift in the relative position between the ultrasound transceiver and the tip of the interventional device. This may happen for example with catheter type interventional devices that bend to squirm to be accommodated by the various bodily structures through which they are inserted into the body of a subject. In another example, the decision to use an ultrasound transducer to track the position of a tip of a interventional device may only be taken once the interventional device has been inserted into the subject, for example, because it is found that the tip of the interventional device has lower than expected visibility due to the subject's anatomy. In a further example, workflows of certain medical procedures may require the insertion of multiple different objects into the interventional device. For example, in crossing a chronic total occlusion, a very stiff crossing guide wire may first be inserted into an ablation catheter to fracture the hard cap of the occlusion, to facilitate laser ablation of the fibrous cap. After cap penetration, a softer crossing wire may be used to cross the rest of the lesion. Positioning an ultrasound transceiver with respect to the tip of each of these wires would allow the clinician to determine the position of the tip of each wire with respect to the opening of the device. Alternatively, during the procedure these different wires could be swapped out for a generic tracking wire whenever the position of the ablation catheter tip needs to be known, and specialty wires then placed back to continue next treatment step. This allows for the development of a single general-purpose tracking wire that can be used to track a multitude of catheter devices.

The receive signals are converted to receive data comprising echo characteristics and the receive data may be processed by a data processor to extract relative position of the transceiver with respect a distal opening of an interventional device.

The inventors have not only realized that certain characteristics of the echo change depending on its relative position with respect to an opening of an interventional device, but also that such changes may be used to derive an indication of the relative position. With this, in turn, a desired relative position may be achieved while the opening is inserted into a subject. Furthermore, once such desired position is achieved and fixed to be maintained during further manipulation of the interventional device, the location of the transceiver may be determined and optionally tracked using an US imaging apparatus to therewith infer the location within the subject of for example the opening of the interventional device. After all, knowing the relative position of the transceiver with respect to the opening allows determining the location of the opening from the location of the transceiver using the relative position. What is more, locations of other parts of the interventional device may also be done by further using relative positions of such other parts with respect to the opening.

The changes in the ultrasound echo's take place over a relatively small position range, so the mechanism provides relatively high degree of precision for the determination of the relative position and a positioning of the ultrasound transceiver with respect to the opening of the interventional device.

In some embodiments the system, device or processor circuit may be configured to determine an indication that represents at least whether the ultrasound transceiver is outside the lumen, inside the lumen, and/or is at an opening, e.g. inside but at an opening, for example, at a tip of the interventional device. In other embodiments the system, device or processor circuit is configured to additionally or alternatively represent a distance of the transceiver to the opening. The indication may be numerical, graphical, auditive or any other suitable indication recognizable by a user. The indication may be provided on a scale or range.

When the ultrasound transceiver is within a threshold distance of an opening of the device, the relative position determined by the data processor may include a location or position or distance of the ultrasound transceiver along for example the length of the lumen, for example a relative distance along the longitudinal dimension of the lumen from the opening of the interventional device. The threshold distance at which it is possible to determine a relative distance from an opening of the interventional device will depend on a number of factors, such as a diameter of the opening, a material of the interventional device, an inside surface roughness of the lumen and an angular transmitting profile of the ultrasound sensor. For example, the threshold distance for an interventional device comprising a glass capillary tube may be twice the diameter of an opening of the glass capillary tube. When the distance between the ultrasound transceiver and an opening of the interventional device is greater than the threshold distance, there may be no significant change in the one or more characteristics of the identified one or more echoes as the ultrasound transceiver is moved further away from the opening.

The one or more characteristics may comprise an amplitude of the identified one or more echoes. The inventors have recognized that the amplitude of the identified one or more echoes is particularly indicative of the relative position of the ultrasound transceiver with respect to an opening of the interventional device.

The one or more characteristics may comprise an amplitude response of the identified one or more echoes. An amplitude response may, for example, be a length of the identified one or more echoes, or a measure of how quickly the identified one or more echoes attenuate. The inventors have recognized that the amplitude response of the identified one or more echoes is particularly indicative of the relative position of the ultrasound transceiver with respect to an opening of the interventional device.

The one or more characteristics may comprise a time delay between an ultrasound transmission and the corresponding echo in the receive signal. The inventors have recognized that the time delay between an ultrasound transmission and the corresponding echo in the receive signal is particularly indicative of the relative position of the ultrasound transceiver with respect to an opening of the interventional device.

When the ultrasound transceiver is inside the lumen, the echo has a large amplitude, is received quickly and attenuates slowly. When the ultrasound transceiver is at an opening of the device, the attenuation of the echo increases. When the ultrasound transceiver is outside the device, the time taken to receive the echo is longer, and the echo has a lower amplitude and is severely attenuated. These characteristics of the echo therefore display distinct differences according to the relative position of the ultrasound transceiver with respect to an opening of the interventional device. The changes in the characteristics of the echo change over a very small position range (of the order of the diameter of the opening of the interventional device), allowing the ultrasound transceiver to be positioned at an opening of a interventional device with a high level of accuracy.

In some embodiments, the data processor may be adapted to determine the relative position of the ultrasound transceiver with respect to the at least one opening by comparing the one or more characteristics of the identified one or more echoes with one or more corresponding reference values.

In this way, the relative position of the ultrasound transceiver may be determined from a single echo received by the ultrasound sensor. The reference values may be corresponding expected values for inside a interventional device, at an opening of a interventional device and/or outside a interventional device for a particular material and size of the lumen of the interventional device.

In some embodiments, the receive signal may comprise a plurality of echoes, and the data processor may be adapted to determine the relative position of the ultrasound transceiver with respect to the at least one opening by comparing the one or more characteristics of a most recent echo of the plurality of echoes with at least one corresponding value for at least one previous echo in the receive signal.

In this way, whether the ultrasound transceiver is inside the interventional device, at an opening of the device or outside the device may be determined for any suitable interventional device, without needing to consider the material and size of the lumen of the interventional device. Comparing a most recent echo with previous echoes also allows a user of the system to monitor the progression of the ultrasound transceiver as it is moved through the interventional device.

According to another aspect of the invention, there is provided a system for determining a relative position of an ultrasound transceiver with respect to an opening of a interventional device comprising a lumen with at least one opening.

The system comprises: an ultrasound transceiver moveable through the at least one opening of the interventional device and configured to transmit and receive ultrasound, and generate a receive signal responsive to received echoes of ultrasound transmissions; and the data processor according to any previously described embodiment.

In some embodiments, the system may further comprise a retrofit device configured to move the ultrasound transceiver through the interventional device.

The retrofit device may be any suitable elongate interventional device, such as a guidewire or a catheter.

By using the retrofit device to move the ultrasound transceiver through the interventional device, the ultrasound transceiver may be added to the lumen of a interventional device while the interventional device is inserted in a subject, or the relative position of the ultrasound transceiver relative to an opening of a interventional device may be adjusted while the interventional device is inserted in a subject.

The ultrasound transceiver may be attached to one end of the retrofit device.

In some embodiments, the system may further comprise a clamp for locking the ultrasound transceiver in a fixed position with respect to the at least one opening of the interventional device.

The clamp may, for example, be attached to a part of the interventional device that is not inside the subject, and may lock the ultrasound transceiver in a fixed position with respect to an opening of the interventional device by clamping the retrofit device that moves the ultrasound transceiver through the interventional device.

The clamp may be used to fix the position of the ultrasound transceiver with respect to the opening of the interventional device responsive to the ultrasound transceiver being determined to be at or close to an opening of the interventional device.

The clamp may be configured to be automatically engaged on receiving feedback from the data processor that the ultrasound transceiver is at a desired relative position with respect to an opening of the interventional device. Alternatively, the clamp may be engaged manually by a user of the system in response to the user determining that the relative position of the ultrasound transceiver determined by the data processor is a desired relative position.

The clamp may be engaged, either automatically or manually, before each ultrasound transmission from the ultrasound sensor, to ensure that the relative position of the ultrasound transceiver with respect to an opening of the interventional device does not change between the transmission and the determination of the relative position. The clamp may then be disengaged in response to the determined relative position not being a desired relative position, in order to allow the ultrasound transceiver to be moved to a new relative position or may remain engaged in response to the determined relative position being a desired relative position.

In some embodiments the device and/or processor circuit may be further configured to control the ultrasound transceiver to transmit the ultrasound and measure the echo to therewith provide the receive signal. It may be further configured to generate the receive data comprising at least part of the receive signal and or one or more echoes. The device may have a controller communicatively coupled to the transceiver and communicatively coupled to the processor circuit, where the controller is capable of providing transceiver drive signals under control of the processor circuit and measure receive signals under control of the processor circuit and provide receive signal and or receive data to the processor.

In some embodiments the processing circuit has a further input for receiving ultrasound image data comprising an ultrasound image of a region of interest of a subject and location data representative of a transceiver location of the ultrasound transceiver within the ultrasound image, wherein the data processor is configured to generate the result data to comprise the ultrasound image and the transceiver location and/or the location of the opening of the interventional device.

According an aspect there is provided a system comprising:
- a device of any one of the claims 1 to 6 comprising the output, and
- a user interface communicatively coupled to the output and adapted to provide the indication to a user such as a caregiver, medical practitioner or subject.

The user is advantageously made aware of the indication via the user interface, preferably there is display as part of the interface that is capable of showing a visualization of the indication in the form of the numeric, graphics or a scale or range.

In some embodiments the system comprising the ultrasound transceiver and the interventional device. These may be connected or disconnected but connectable to the device. The device may be a console of an ultrasound system.

In some embodiments of the system the ultrasound transceiver is moveable through the opening, the system comprising a retrofit device adapted to move the ultrasound transceiver through the opening of the interventional device. For example, the ultrasound transceiver can be attached to one end of the retrofit device while another end may be manipulated by a user. Additionally, the ultrasound transceiver and, if present, the retrofit device may be removable from the interventional device.

In some embodiments the system further comprising a locking mechanism for locking the ultrasound transceiver in a fixed position with respect to the opening. Once a relative position is fixed, further manipulation of the ensemble of transceiver and interventional device maybe done without loss of positional information. The locking mechanism may be releasable temporarily should a redetermination of a relative position by repeating of the necessary steps be needed.

In some embodiments the system comprises or consists of an ultrasound imaging system comprising an ultrasound imaging probe adapted to transmit and receive ultrasound for generating an ultrasound image of a region of a subject and wherein the system or device is further configured to generate an indication of a location of the ultrasound transceiver in the ultrasound image based on an ultrasound based interaction of the ultrasound transceiver with the ultrasound imaging probe.

The ultrasound-based interaction may comprise use of:
a characteristic of a beam transmitted by the ultrasound imaging probe and received by the ultrasound transceiver and a time between transmission and reception, and/or
a characteristic of a beam transmitted by the ultrasound transceiver and received by the ultrasound imaging probe, and a time between transmission and reception.

In some embodiments, the system may further comprise an ultrasound imaging probe configured to transmit and receive ultrasound imaging beams, and the data processor may be further adapted to determine a position of the ultrasound transceiver relative to the ultrasound imaging probe based on: a characteristic of a beam transmitted by the ultrasound imaging probe and received by the ultrasound transceiver and a time between transmission and reception, and/or a characteristic of a beam transmitted by the ultrasound transceiver and received by the ultrasound imaging probe, and a time between transmission and reception.

In this way, the position of the ultrasound transceiver in the subject, and therefore the position of the tip of the interventional device, can be determined. The position of the ultrasound transceiver in an imaging frame of the ultrasound imaging probe may be determined from the characteristic of the beam, for example, an angle of the beam, and the distance between the ultrasound transceiver and the ultrasound imaging probe may be determined from the time of flight of the beam.

The beam used to determine the position of the ultrasound transceiver relative to the ultrasound imaging probe may be a largest received amplitude beam of a plurality of beams transmitted by the ultrasound imaging probe and received by the ultrasound sensor, or transmitted by the ultrasound transceiver and received by the ultrasound imaging probe.

This recognizes that the beam that is received with the largest amplitude is the closest beam to a line between the ultrasound transceiver and the ultrasound imaging probe, and that angle and time of flight of the closest beam can be used to most accurately determine the position of the ultrasound sensor.

In some embodiments, the system may further comprise: an image generation processor adapted to: generate at least one ultrasound image of a region of the subject from ultrasound imaging beams received by the ultrasound imaging probe, wherein the region of the subject includes the ultrasound sensor; and provide an indication of the position of the ultrasound transceiver in the at least one ultrasound image based on the determined position of the ultrasound transceiver relative to the ultrasound imaging probe; and a display device adapted to: obtain, from the image generation processor, and display the at least one ultrasound image and the indication of the position of the ultrasound sensor.

Displaying an indication of the position of the ultrasound transceiver in an ultrasound image allows a clinician to see the position of the tip of the interventional device in the subject. This allows the clinician to determine whether the interventional device is in the desired position for a medical procedure, and, if not, the direction in which the device should be moved.

The system may further comprise a interventional device comprising a lumen with at least one opening. The relative position of the ultrasound transceiver may be determined with respect to the at least one opening of the interventional device.

According to another aspect of the invention, there is provided a computer-implemented method for determining a relative position of an ultrasound transceiver with respect to an opening of a interventional device comprising a lumen with at least one opening, wherein the ultrasound transceiver is configured to be moveable through the at least one opening. The features of such method may be defined along analogous features of the embodiments defined for the system, device and processor circuit aspects and provide similar or the same advantages.

The computer-implemented method comprises: obtaining a receive signal from the ultrasound sensor, wherein the receive signal is responsive to received echoes of ultrasound transmissions; identifying one or more echoes of an ultrasound transmission in the receive signal; and determining a relative position of the ultrasound transceiver with respect to the at least one opening based on one or more characteristics of the identified one or more echoes.

The one or more characteristics may comprise at least one of: an amplitude of the identified one or more echoes, an amplitude response of the identified one or more echoes, and/or a time delay between an ultrasound transmission and the corresponding echo in the receive signal.

According to another aspect of the invention, there is provided a computer program product comprising computer program code means which, when executed on a computing device having a processing system, cause the processor system, device or system to perform the steps of any previously described method. The program product may be stored on a computer readable medium or may be carried on a computer readable carrier such as a signal. The computer readable medium may be a non-transitory computer readable medium.

These and other aspects of the invention will be apparent from and elucidated with reference to the embodiments described hereinafter.

### BRIEF DESCRIPTION OF THE DRAWINGS

For a better understanding of embodiments of the invention, and to show more clearly how they may be carried into effect, reference will now be made, by way of example only, to the accompanying schematic drawings, in which:
FIG. 1 shows a diagram of an ultrasound system;
FIG. 2 shows an ultrasound transceiver inside a interventional device;
FIG. 3 shows a graph of a received echo of an ultrasound pulse transmitted by the ultrasound transceiver of FIG. 2;
FIG. 4 shows an ultrasound transceiver at an opening of the interventional device of FIG. 3;
FIG. 5 shows a graph of a received echo of an ultrasound pulse transmitted by the ultrasound transceiver of FIG. 4;
FIG. 6 shows an ultrasound transceiver positioned outside the interventional device of FIG. 2;
FIG. 7 shows a graph of a received echo of an ultrasound pulse transmitted by the ultrasound transceiver of FIG. 6;
FIG. 8 shows 6 graphs each one of pulse-echo data collected from a doppler flow wire (Volcano FloWire^{®}) having its ultrasound transceiver positioned at different positions with respect to an opening of a glass capillary tube;
FIG. 9 shows 8 graphs each one of pulse-echo data collected from a doppler flow wire (Volcano FloWire^{®}) having its ultrasound transceiver positioned at different positions with respect to an opening of a stainless steel hollow needle;
FIG. 10 shows a diagram of some embodiments of a system for determining a relative position of an ultrasound transceiver with respect to an opening of an interventional device;
FIG. 11 shows a processor circuit for a system according to embodiments; for example for a system of FIG. 10.
FIG. 12 shows at least a part of a computer-implemented method according to some embodiments.
FIG. 13 show a flow diagram of a method according to some embodiments.
FIG. 14 shows a display of an ultrasound image and an indication of a position of an ultrasound transceiver.

### DETAILED DESCRIPTION OF THE EMBODIMENTS

The invention will be described with reference to the Figures.

The detailed description and specific examples and embodiments of the systems and methods, are intended for purposes of illustration only and are not intended to limit the scope of what is claimed. These and other features, aspects and advantages of the systems and methods claimed and disclosed herein will become better understood from the following description, appended claims, and accompanying drawings. It should also be understood that the same reference numerals are used throughout the Figures.to indicate the same or similar parts.

According to a proposed concept, there is provided a data processor and a method for determining a relative position of an ultrasound transceiver with respect to an opening within a hollow portion (which may be referred to as a lumen) of an interventional device. The ultrasound transceiver is movable with respect to the opening and preferably through the opening such that it may be relocated from a position within the hollow portion to a position in which it is at least partially outside of the hollow portion. Ultrasound transmissions from such ultrasound transceiver are reflected off the lumen wall to provide echoes of such transmissions. The ultrasound transceiver receives one or more echoes of the ultrasound transmission and generates a receive signal that comprises or at least partially represents the one or more received echoes. The controller comprises a processing circuit with an input configured to receive the receive signal and a data processor of the processor circuit is configured to identify the one or more echoes in the receive signal, and to determine a relative position of the ultrasound transceiver with respect to the at least one opening based on the one or more characteristics of the identified one or more echoes.

Some embodiments of such controllers may, for example, be employed in Ultrasound systems (for example Ultrasound imaging systems) for tracking a tip of a interventional device that has been retrofitted with an ultrasound transceiver. For example, once the relative position of the ultrasound transceiver with respect to the opening of the interventional device has been fixed such that it is known and the transceiver is capable of being located by an ultrasound imaging system, the system may be used to guide the interventional device to a desired anatomical region using ultrasound imaging.

Some embodiments may be at least partly based on the realization that certain characteristics of an echo of a ultrasound transmission are different according to whether the transmission is from an ultrasound device inside the lumen of a interventional device, at an opening of the interventional device or outside the interventional device.

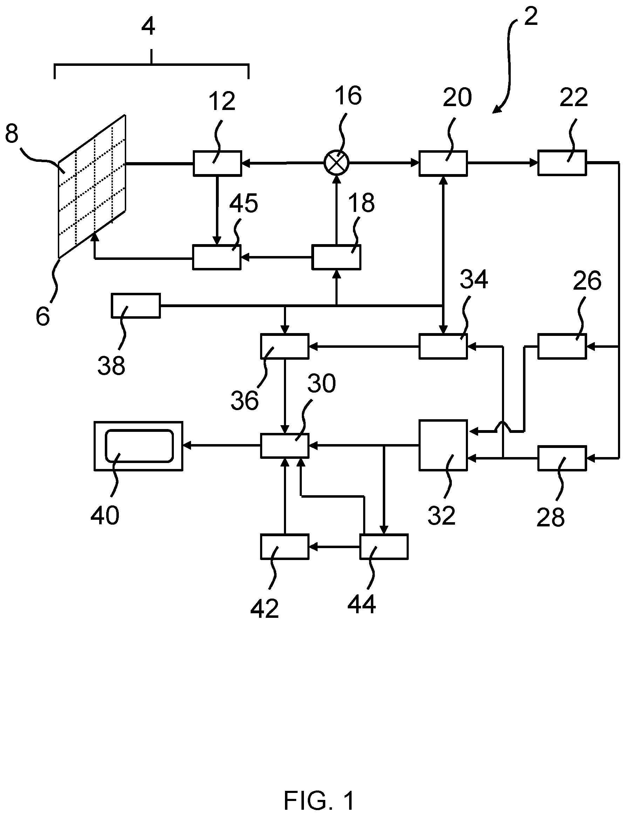

The general operation of an ultrasound system will first be described, with reference to FIG. 1.

In some embodiments the system comprises an ultrasound probe 4 which has a transducer array 6 for transmitting and receiving ultrasound. The transducer array 6 may comprise CMUT transducers; piezoelectric transducers, formed of materials such as PZT or PVDF; or any other suitable transducer technology. In this example, the transducer array 6 is a two-dimensional array 8 of transducer elements. The array 6 is capable of scanning either a two-dimensional plane or a three-dimensional volume of a region of interest of, for example a body part of a subject or patient. In another example, the transducer array may be a one-dimensional array for scanning a two-dimensional plane of such region.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one-dimensional line of transducers or a two-dimensional patch of transducers within the larger array. In transmit mode, the focusing and steering of the ultrasound beam generated by the array, or a sub-aperture of the array, are controlled as described below.

Upon receiving the backscattered ultrasound echo signals from the region of interest of a subject, the received signals undergo receive beamforming (as described below), in order to align the received signals, and, in the case where a sub-aperture is being used, the sub-aperture is then shifted, for example by one transducer element. The shifted sub-aperture is then activated, and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to consider the characteristics of the transmission beamformer. In FIG. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two-dimensional (2D) sector-shaped format, or a pyramidal three-dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

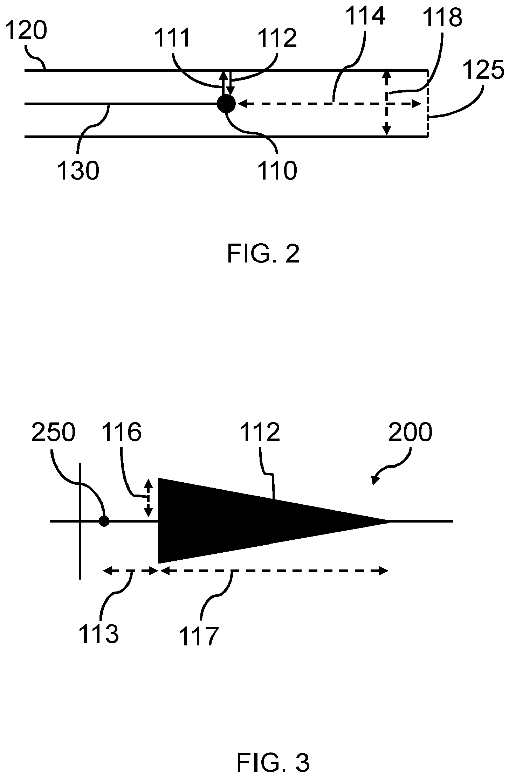

FIG. 2 illustrates an ultrasound transceiver 110 inside an interventional device 120 of which only a hollow portion (which may also be referred to as a lumen or interventional device lumen) is shown. The interventional device 120 typically is a device suitable for insertion into a subject a region of interest of a subject or an anatomical region of a subject, and for example may comprise, but is not limited to an endoscope, catheter or needle. The hollow portion of such interventional devices can comprise an opening 125, for example at a distal end of the interventional device. In FIG. 2, the ultrasound transceiver 110 is at a distance 114 from the opening 125. The ultrasound transceiver 110 can be moved through the interventional device 120 by a retrofit device 130. It may be part of the retrofit device. The retrofit device 130 may be another elongate interventional device such as a guidewire or a catheter with a smaller diameter than the inner diameter of the lumen of the interventional device 120.

The ultrasound transceiver 110 can transmit an ultrasound signal such as for example a pulse 111 and is also capable of receiving an ultrasound echo 112 of the transmitted ultrasound pulse 111. FIG. 3 shows an exemplary echo comprising signal in the form of a graph 200 of ultrasound intensity or amplitude (vertical axis or y-axis) received by the transceiver 110 as a function of time (horizontal or x-axis) after the transceiver has transmitted an ultrasound pulse 112 at time 250 when the ultrasound transceiver 110 is in the position at distance d (114 in FIG. 2) from the opening within the device 120. In this position, the transmitted pulse 111 is reflected off a wall of the lumen of the interventional device and continues to be reflected back and forth between walls of the lumen several times before eventually attenuating by among others escaping through the opening 125. As a consequence, the ultrasound echo 112 in graph 200 as represented schematically by the black triangle follows the transmission instance 250 with a relatively short delay time 113 after the pulse 111 has been transmitted and has a relatively high intensity as represented by a relatively large initial amplitude 116 that decays or attenuates relatively slowly over a decay time 117.

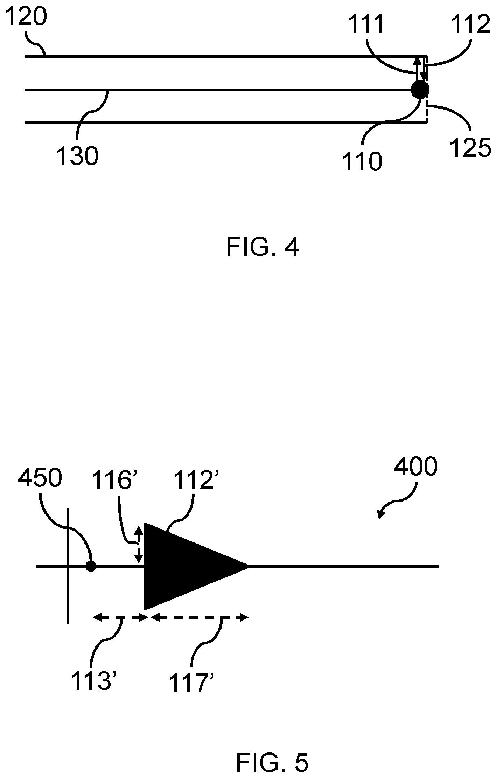

FIG. 4 illustrates the same part of the interventional device 120 as shown in FIG. 2, but with the ultrasound transceiver 110 moved further along the interventional device 120 to be positioned at, or close to, the opening 125. Thus, in this case the distance 114 is zero (the distance it is not specifically indicated to increase clarity). The ultrasound transceiver 110 may be considered to be close to the opening 125 when the distance 114 between the ultrasound transceiver 110 and the opening 125 is less than a diameter 118 (see FIG. 2) of the opening 125.

In some embodiments the ultrasound transceiver includes an integrated ultrasound transmitter and receiver. In such case the same element is driven to convert the drive signals into the transmitted ultrasound and to generate the receive signals from received ultrasound. To be able to simplify the determination of the delay time between transmission and reception the ultrasound may be provided pulse form with a relatively short pulse time duration and the controller may be configured to generate a drive signal resulting in such ultrasound pulse. For example, pulse or drive signal waveforms may be substantially rectangular. However, other waveforms such as sine or triangular may be used. Preferably the time duration is shorter than the shortest delay time such as the delay time 112, but this is not necessary per se.

Additionally, or alternatively, the ultrasound transceiver comprises an ultrasound transmitter that can be separately driven from the ultrasound receiver in the transceiver. This allows to transmit and receive ultrasound simultaneously and/or continuously. In such case, preferably the receiver is positioned on the retrofit device such that when both the transmitter and receiver are located within the hollo portion of the interventional device, the receiver is nearer the opening of the interventional device than the transmitter and/or moves out of the opening before the transmitter. In this way the receiver signal echo may provide a more accurate indication for the location of the opening of the interventional device.

FIG. 5 shows a time dependent ultrasound intensity graph 400 similar to that shown in FIG. 3, but now for an ultrasound echo received when a pulse 111 is transmitted at time 450 by the ultrasound transceiver 110 when located at the position shown in FIG. 4. In this position, the wall of the interventional device is still nearby the transceiver, it is in fact at comparable distance as that shown for situation of FIG. 2. Hence, the echo 112' is received with a time delay 113' that is comparable to the time delay 113. Also, the initial intensity as represented by the amplitude 116' is comparable to amplitude 116 of FIG. 3, although it may be somewhat smaller due to a larger part of the transmitted signal having escaped through the opening directly after transmission. The location of the transceiver at the opening also may cause less back and forth reflection as with the situation of FIG. 2 and the echo 112' consequently may have a shorter decay time 117' compared to decay time 117, i.e. the echo 112'may attenuate faster than echo 112.

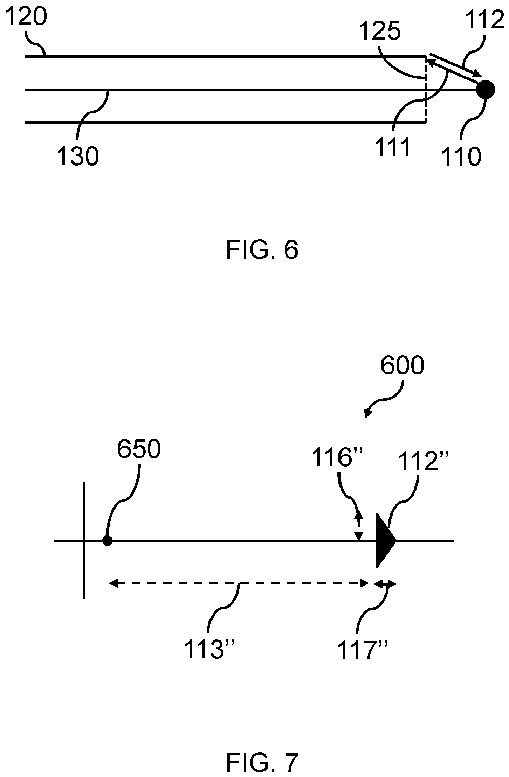

FIG. 7 shows yet another time dependent ultrasound intensity graph 600 similar to that shown in FIGS. 3 and 5, but now for an ultrasound echo received when a pulse 111 is transmitted at time 650 by the ultrasound transceiver 110 when moved beyond the opening at the position shown in FIG. 6. In this case the transceiver is outside of the device 120. In this position, the transmitted pulse 111 must travel further to be reflected off a wall of the lumen, the echo must travel further to be received by the ultrasound transceiver 110, and much of the acoustic energy from the transmitted pulse 111 escapes, before it can be reflected and/or received again. Accordingly, the echo 112" is now received with a relatively large delay time 113", i.e. a delay time 113" that is generally longer than the delay times 113 and 113' and smaller initial intensity as represented by a smaller initial amplitude 116". Also, the back and forth bouncing of the wall of the pulse is greatly reduced and this results in a decay time 117" that is generally shorter than decay times 117 and 117', such that the initial amplitude 116" attenuates more quickly, than those of the echoes 112 and 112'.

FIGS. 2 to 7 show that one or more of characteristics of the received echo, such as the delay time, the initial amplitude and decay time are different according to where the ultrasound transceiver 110 was located with respect to the opening of the device 120 during transmission of an ultrasound signal, for example according to when the transceiver is inside the lumen of the interventional device 120, at an opening 125 of the interventional device 120, or outside the interventional device 120. The initial amplitude of the received echo 112 may slowly decrease as the ultrasound transceiver 110 moves within the device 120 towards the opening but decreases more with increased distance from the opening when outside of the device. In accordance with such movements, the decay time of the received echo 112 decreases as the ultrasound transceiver 110 moves from inside the interventional device 120 to approach the opening 125, and eventually passes the opening and protrudes through the opening outside of the device 120. A time delay between a transmitted pulse 111 and a corresponding received echo 112 increases as the ultrasound transceiver 110 moves out of and away from the interventional device 120.

The received ultrasound echo can therefore be used to determine the relative position, e.g. the distance d of the transceiver with respect to the opening, or a distance representative of such distance of the ultrasound transceiver 110 with respect to the opening 125 of the interventional device 120. Additionally, or alternatively it may be used to determine at least whether the ultrasound transceiver 110 is inside the lumen of the interventional device 120, at or close to, an opening 125 of the interventional device 120, or outside the interventional device 120. In some embodiments it may be used to determine a distance or position of the ultrasound transceiver from an opening of the interventional device when the ultrasound transceiver is inside the lumen and within a threshold distance (measured inside the tube) of the opening. The threshold distance may be user defined, fixed or otherwise set.

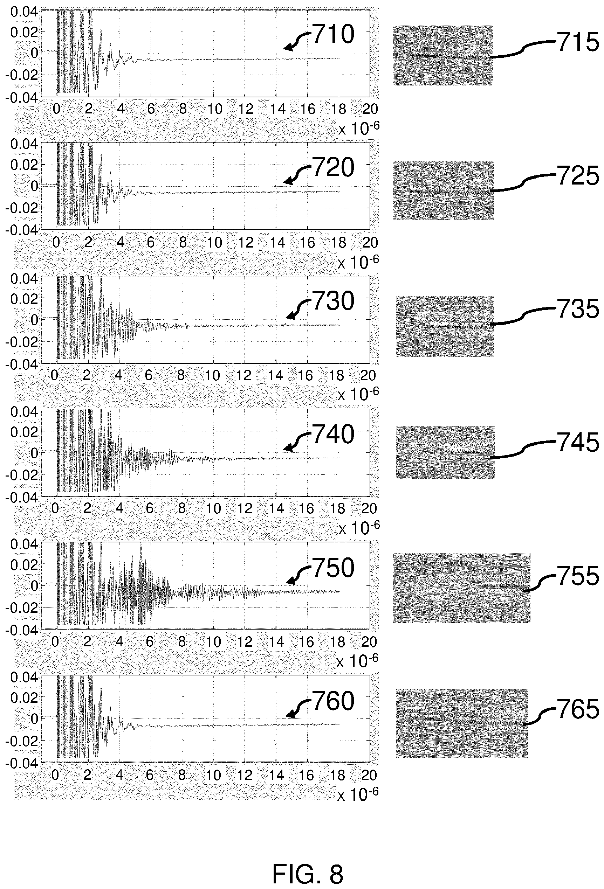

FIG. 8 illustrates a set of graphs 710 to 760 showing ultrasound pulse-echo's measured using a doppler guidewire (in this case a Volcano FloWire^{®}) inside, at an opening of, and outside a glass capillary tube, to demonstrate proof of concept. In this case the glass tube is to resemble the interventional device 120 and the FloWire^{®} is to serve as an example of a retrofit device 130. The FloWire^{®} is a 0.014" diameter wire with a donut shaped 12 MHz piezoelectric element at the tip as the ultrasound transceiver 110 and is connected to a Panametrics NDT 5800 pulser/receiver for operating the transceiver. The glass capillary tube and FloWire^{®} are submerged in water to mimic a medium of a subject.

The graphs 710 to 760 are accompanied by photographs 715 to 765 showing the position of the tip of the FloWire^{®} with respect to an opening of the glass capillary tube corresponding to each graph. The tip of the wire comprises the ultrasound transceiver.

Due to hardware limitations of the setup and for example the electrical driver of the ultrasound transducer, the first three microseconds of each graph 710 to 760 are dominated by ringdown artifacts and amplifier saturation recovery. However, the echo data received after three microseconds in each graph show clear differences according to whether the FloWire^{®} tip is inside, at or close to an opening of, or outside the glass capillary tube. The differences would be more pronounced, and artifacts reduced with improved electronics for the driving of the transceiver. The concept is however clearly shown with the measurement data. In embodiments of the controller and system, the controller may be configured to filter the receive signal to select a part of the signal that is representative of the ultrasound echo. It may thus be configured to remove ringdown effects.

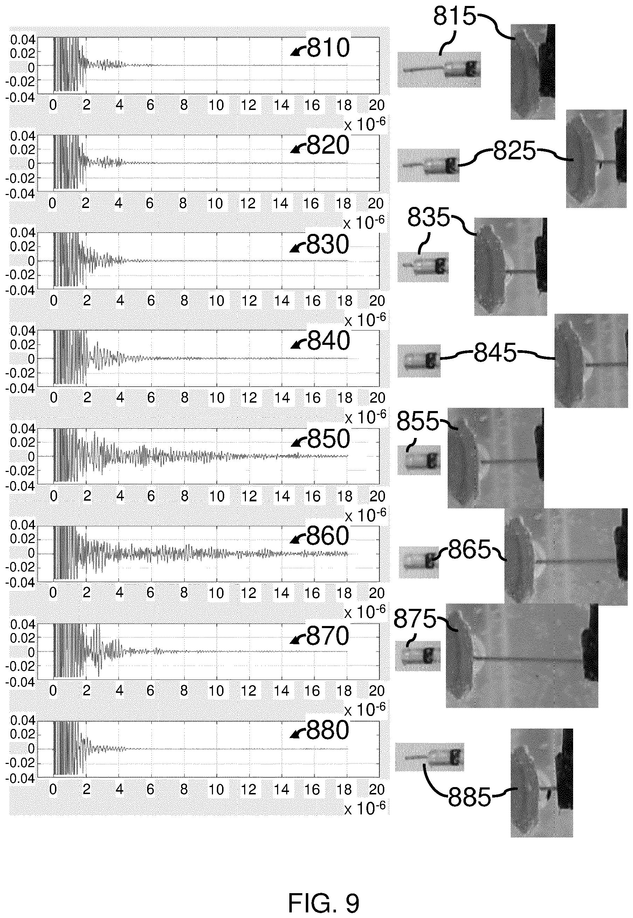

FIG. 9 illustrates a set of graphs 810 to 880 showing pulse-echo data collected from a doppler guidewire (e.g. a Volcano FloWire^{®}) as the retrofit device 130 positioned within, at an opening of, and outside a stainless steel needle (the interventional device), to demonstrate further proof of concept. The apparatus is identical to that used to collect the data shown in FIG. 8, except that the glass capillary tube has been replaced by the stainless-steel needle.

The graphs 810 to 880 are accompanied by photograph pairs 815 to 885 showing the position of the tip of a FloWire^{®} identical to that described herein before with respect to a first opening of the stainless-steel needle corresponding to each graph. The needle now serves as an example of the interventional device while again the wire serves as an example of a retrofit device. Since the tip of the FloWire^{®} cannot be seen when it is inside the stainless-steel needle, the photograph pairs show both ends of the stainless steel needle, allowing the amount of FloWire^{®} protruding from both openings of the stainless steel needle to be seen.

Again, the echo's measured in the graphs 810 to 880 show clear differences according to whether the FloWire^{®} tip is inside, at or close to an opening of, or outside the stainless-steel needle.

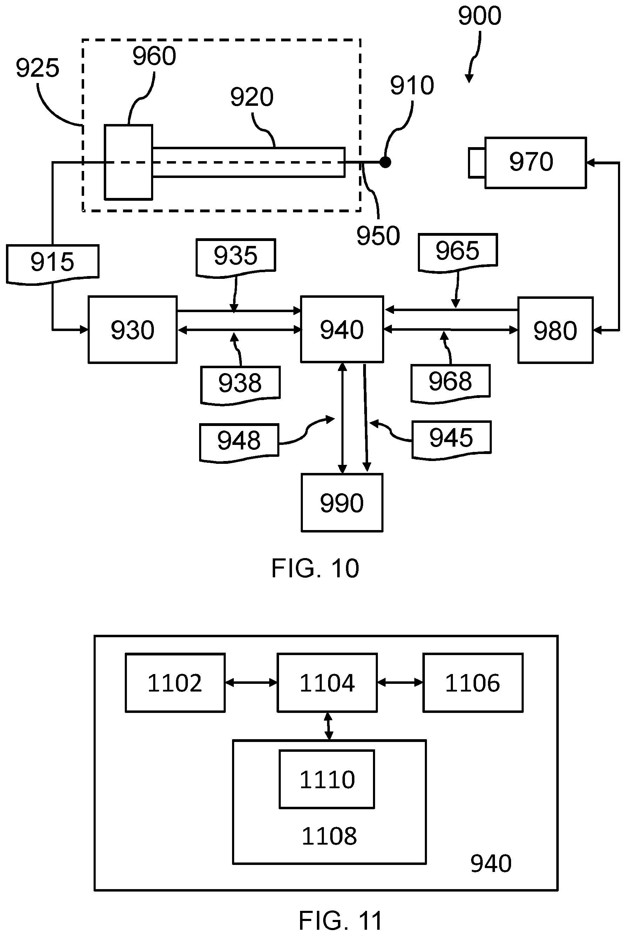

FIG. 10 illustrates a diagram of a system 900 for determining a relative position of an ultrasound transceiver 910 with respect to an opening within a hollow portion 920 of an interventional device 925, according to embodiments of the invention. The interventional device 925 comprises a lumen 920 as the hollow portion. The interventional device may for example have the hollow portion as described with reference to FIG. 2. The lumen is part of the device that is usually for insertion into a region of interest of a subject where the distal opening 125 is also inserted into this region. The interventional device in this example also has a proximal opening at the other end of the lumen s that a retrofit device may be slidably moved within the interventional device. The interventional device 925 may be a interventional device suitable for insertion into a subject, such as a catheter, or a needle of a syringe etc.

The system 900 comprises the ultrasound transceiver 910 as part of a retrofit device 950 to transmit and receive ultrasound radiation. The retrofit device may for example be a doppler guide wire such as the FloWire^{®} referred to herein above or any other device that can move through the hollow portion and that includes the transceiver. In FIG. 10, the retrofit device 950 can be seen to be deployed in the lumen such that it enters the lumen at the proximal opening and slightly extends with its tip outside of the device 920 at the distal opening. The ultrasound transceiver 910 is moveable through the distal opening 125 of the interventional device for example through manipulation of the retrofit device 950 which is slidably disposed within the lumen of the interventional device and can be removed entirely if needed by retracting the retrofit device through distal opening and subsequently the proximal opening.

The system comprises a device that includes the processor circuit 940. Additionally, the system and preferably as part of the device it may comprises controller 930. The device may be for generating an indication of the relative position as described for example herein.

Once the transceiver controller 930 has determined the relative position of the ultrasound transceiver 910 with respect to the opening of the interventional device 920, the data processor may send the determined relative position, or an indication of the relative position, to the display device 940. In this way, a user of the system 900 may be provided with information that allows the user to adjust the relative position of the ultrasound transceiver 910 to achieve a desired relative position with respect to an opening of the interventional device 920.

The controller 930 may comprise an electrical signal generator and/or an electrical signal measurer configured to process, provide and/or detect electrical signals, including e.g. voltages and currents. It is communicatively connected to the transceiver for example through the retrofit device 950 via wiring or via wirelessly enabled communication as known in the art. In this way, the controller 930 is capable of providing the transceiver with signals to have it transmit ultrasound and/or receive the receive signals form the transceiver generated by ultrasound echo's. The transceiver controller has the necessary electronics, such as for example signal generators and the like as well as signal processors such as ADC etc., to generate the transceiver drive signal and to measure or collect the transceiver echo signal. The transceiver controller 930 is further connected via a suitable data link to a processor circuit 940, which in turn is communicatively connected with a user interface 990. The user interface preferably includes one or more input devices such as: keyboard, touchscreen, mouse, joystick microphone for a user to provide commands to the system. The processor circuit and controller are therewith capable of intercommunicating data, signals and/or communicating instructions 948. For example, the processor circuit is configured to control the controller for in turn operating the transceiver. This may be done possibly as a consequence of commands 948 provided by a user to and communicated by the user interface 990 to the processor circuit. The user interface furthermore comprises one or more audio or video/image output devices such as a speaker and/or display for the processor circuit and the system to output results 945 generated by the processor circuit 940, where the results comprise an indication of location of the transceiver with respect to the interventional device distal opening.

FIG. 11 is a diagram of a processor circuit 940, according to embodiments of the present disclosure. The processor circuit 940 may be implemented in the device and/or system and/or for example partly in the retro device. The processor circuit can carry out one or more steps described herein. The processor circuit 940 comprising, a processor 1104 communicatively connected via suitable data busses with an input 1102 configured to receive data from the controller 930 and an output 1106 configured to provide processor results to the user interface 990. The processor circuit also includes connections for communicating with the controller 930 to have it operate the transceiver under control of the processor circuit. In embodiments these connections and the input 1102 may be part of one integrated I/O interface, but this is not necessary. Similarly, the processing circuit preferably also includes connections for communicating with the user interface 990 and again these connections may be part of a further integrated I/O interface that also includes the output 1106, but this is again not necessary. The various inputs or outputs may be comprised in a communication module that can include any electronic circuitry and/or logic circuitry to facilitate direct or indirect communication of data between the processor circuit, the controller or transceiver and/or the user interface including for example the display. In that regard, the communication module can be an input/output (I/O) device. In some instances, the communication module facilitates direct or indirect communication between various elements of the processor circuit and/or the system.

The system, device or processor circuit makes use of a processor to perform data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions. The processor may include a central processing unit (CPU), a digital signal processor (DSP), an ASIC, a controller, an FPGA, another hardware device, a firmware device, or any combination thereof configured to perform the operations described herein. The processor may also be implemented as a combination of computing devices, e.g., a combination of a DSP and a microprocessor, a plurality of microprocessors, one or more microprocessors in conjunction with a DSP core, or any other such configuration.

The processing circuit 1100 may, and preferably has, a memory 1108 for storing (volatile memory) or that stores (permanent memory) instructions 1110 for processing the received data 915 from the controller to determine the indication of the location of the transceiver as described herein. The memory is communicatively connected via suitable data busses with the data processor.

The memory may include a cache memory (e.g., a cache memory of the processor), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory includes a non-transitory computer-readable medium. The non-transitory computer-readable medium may store instructions or code that can be run by the processor. For example, the memory, or non-transitory computer-readable medium may have program code stored or recorded thereon, the program code including instructions for causing the system, device, processor circuit, or one or more components of the processor circuit such as the data processor, to perform operations described herein. For example, the processor circuit can execute operations described with reference to for example the methods described herein. Instructions may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory, with the code recorded thereon, may be referred to as a computer program product.

The ultrasound transceiver 910 is configured to receive from the controller 930 drive signals that it converts into ultrasound transmitted and to generate receive signals 915 from received ultrasound which receive signals can be collected by the controller. Thus, in embodiments, the receive signals 915 may comprise the echo of the interventional device because of transceiver transmitted ultrasound when the transceiver is used in conjunction with the interventional device as explained herein. In such case the receive signal 915 may comprise echoes of ultrasound transmissions by the transceiver 910 that are indicative of the relative position, i.e. the position of the transceiver with respect to the proximal opening of the interventional device.

The receive signals, or parts thereof, that comprise echo information indicative for the relative position are then sent as data in a suitable data format to the processor circuit. The generation of the data with a suitable data format may entail signal transformation by the controller from analog to digital for example and/or may comprise other processing. However, any data format that can be used by the processor circuit will work.

The processor circuit 940 therefore comprises an input 1102 for receiving the data comprising the echo information and the data processor is configured to process the received data to determine a relative position of the ultrasound transceiver 910 with respect to an opening of the interventional device 925, using the echo information in the received data. In embodiments the data processor is further configured to generate a result comprising an indication of the position which result can be output via the output 1106. In embodiments the user interface includes one or more devices, such as for example a display, for providing the result to a user such as e.g. patient or medical practitioner.

The data 935 received via the input 1102 may comprise and/or be representative of one or more entire receive signals each one generated after a transmit pulse. In such case all echo information is available to the data processor. Alternatively, or additionally, the data may comprise only time segments of such receive signals such that it includes only certain time segments of echo's as echo information. In such case only partial echo information may be available to the data processor such that location can still be determined while data processing may be reduced. In another example the data comprise as the echo information only one or more characteristics of the echo in the receive signal as will be further explained hereinafter. The one or more characteristics may comprise for example one or more of the delay time of the echo, the initial amplitude of the echo and a decay time of the echo.

The data processor may be configured to identify one or more ultrasound echoes in the data 935. Methods for such identification will be apparent to the skilled person and may for example involve identifying one or more sections in the receive signal 915 comprised within the data in which sections an amplitude of the receive signal 915 is above a threshold such as for example a fixed, user determined, or automatically (by the processor circuit or system) set threshold. This is advantageous when the data comprises part of or an entire receive signal in the form of electrical signal transients.

The one or more characteristics of the identified one or more echoes used to determine a relative position of the ultrasound transceiver 910 may comprise at least one of an amplitude of the identified one or more echoes, an amplitude response of the one or more identified echoes, and/or a delay time between an ultrasound transmission and the corresponding echo in the receive signal, and/or some other suitable one or more characteristics of the identified one or more echoes that is indicative of the relative position. An amplitude response or decay time of the identified one or more echoes may, for example, be a length of the identified one or more echoes or a measure of how quickly the identified one or more echoes attenuate. As described above, these characteristics may provide an indication of whether the ultrasound transceiver is inside, at or close to an opening of, or outside the interventional device 920.

The processor circuit or processor is configured to extract one or more echo characteristics from the received data 935 which characteristics are indicative of the relative position of the transceiver. In embodiments such characteristics comprise parts of the echo signal or the entire echo signal or an echo amplitude, initial echo amplitude, echo period, echo amplitude decay rate and/or echo delay time. These characteristics have been defined herein before.

In some embodiments, the data processor 930 is configured to determine the relative position by comparing the one or more echo characteristics with one or more corresponding reference values. These reference values may be determined via suitable calibration procedures conducted with the interventional device and the ultrasound transceiver external to the region of interest of a subject, in a model region of interest that resembles that of a real subject or simply with the interventional device and transceiver outside the region of interest. In such calibration the reference values may be determined and set according to predefined well-known locations of the transceiver with respect to one or more openings of the interventional device. Such reference values correlated to relative positions may be stored in a lookup table to be later retrieved by the data processor and used for the location determination after identifying a certain characteristic and comparing it to the data stored in the lookup table. The lookup table may be stored in a memory such as the memory 1110 for use by the data processor.

For example, one or more of the echo characteristics comprised within the received data may be compared with a corresponding reference value that is expected for the ultrasound transceiver when located at an opening (such as for example the distal opening) of a interventional device. In such case the data processor may be configured, for example, to determine that the ultrasound transceiver is at or close to the opening when a particular echo characteristic in the received data is of substantially the same value as the reference value, i.e. the measured value form the receive data is not substantially different from the reference value. For example, amplitudes may have the same or similar magnitude, decay rates may be the same or similar so that decay profiles are the same or similar, characteristic time periods as mentioned herein before may be of equal or similar duration etc. For example, the echo period may be used for this. Furthermore, the data processor may be configured, for example, to determine that the ultrasound transceiver is inside the interventional device when a particular echo characteristic in the received data is different from the reference value. For example, the difference may be such that the echo period is longer in such case. Also, in such case, an amplitude decay rate may be lower. The initial amplitude of an echo in the received data may be somewhat higher in such case.

Alternatively, or additionally, the data processor may be configured to determine that the ultrasound transceiver is outside the interventional device when a particular echo characteristic in the received data is different from the reference value. For example, the difference may be such that the echo period is shorter in such case. Also, in such case, an amplitude decay rate may be higher and/or the initial amplitude may be lower and/or the delay time may be longer.

A threshold may be set for these differences (or similarities) in order to determine when the differences or similarities are to be regarded as significant or substantial. This may be used to set for example an accuracy of location determination.

Herein above the reference value was described to be related to the situation when the transceiver is at or close to the opening. However, other reference locations can be used alternatively or additionally. Multiple of such reference values for a characteristic may be sued for the comparison. In embodiments the data processor may be configured to determine a distance metric indicating the relative location. For example, the magnitude of a particular difference between corresponding measured and reference characteristics can be translated into a value for such metric. In an example the calibration is performed to provide a plurality of reference values each one obtained at a different known preset relative distance. The plurality of reference value and distance data pairs may serve as calibration data that can be used by the data processor to compare measured characteristics and retrieve a distance location from that comparison. Such may be done through for example regression or the like. The calibration data may be stored as such or in the form of a trend or regression line in a memory for use by the data processor. The echo period for example may be used for this purpose.

In these embodiments, the one or more reference values used may depend on a material and/or design (for example, size in the form of a diameter) of the interventional device tube 920, and the data processor 934 may be further adapted to receive a user input (not shown in FIG. 10) corresponding to such material and/or size of the interventional device 920. Other characteristics associated with particular choices of retrofit device and interventional device that are of influence on the reference values may also be provided through user input. Alternatively, or additionally, such values may be retrieved via predetermined calibration data (as described herein before), which must be uploaded in the processing circuit or other memory for access by the circuit and data processor. Yet in another alternative before use of the system a calibration run is performed to generate the calibration data.

In other embodiments, the received data 935 comprises echo characteristics of a plurality of echoes, and the data processor is configured to determine a relative position by comparing one or more of the echo characteristics of a first one of the plurality of echo's with one or more corresponding echo characteristics of at least one other one of the plurality of echo's where the at least one other one is recorded previous to the first one. In other words, the data processor may determine the relative position by detecting a change in the one or more characteristics.

In the embodiments and examples described hereinbefore, the determination may be performed periodically or even real time (with calculation time delay). An indication of such relative location determination whether only in terms of inside/outside/at or close to an opening or with a distance metric as described herein above may additionally be provided periodically or real time (instantly after determination) to a user. This will allow a user to follow the effect of his actions when manipulating the transceiver (via the retrofit device) to move it towards a desired relative position. Thus, the user may be able to follow how the ultrasound transceiver is moved through the lumen 920 up to the desired position. In other words, as an ultrasound transceiver inside an interventional device is moved closer to an opening of the interventional device, more acoustic energy from an ultrasound transmission from the ultrasound transceiver escapes through the opening. A decrease in a length of an echo, or a faster attenuation of the echo, (or any other characteristic of the echo that is dependent on the movement (see herein before) when compared to a preceding echo, may therefore indicate that the ultrasound transceiver inside the interventional device is approaching the opening.

In another example, the data processor 940 may determine that the ultrasound transceiver 910 has passed through an opening of the interventional device 925, and is now outside the interventional device, by detecting a change in a delay time between an ultrasound transmission corresponding to a most recent echo in the receive signal 915 and thus the receive data 935 when compared to a delay time of an ultrasound transmission corresponding to a preceding echo in the receive signal 915 and the receive data 935. As an ultrasound transceiver moves out of the interventional device and away from the opening, an ultrasound transmission from the ultrasound transceiver must travel further to be reflected off the internal wall of the interventional device and must travel further to be received again by the ultrasound transceiver. An increase in the delay time, may therefore indicate that the ultrasound transceiver is protruding further out of the opening of the interventional device.

In these embodiments, at least one of the plurality of echoes in the receive signal 915 and receive data 935 may have been received while the ultrasound transceiver 910 had a known relative position with respect to an opening of the interventional device 925. For example, the earliest echo in the receive signal 915 may have been received while the ultrasound transceiver is known to be inside the lumen of the interventional device 925.

The data processor may be configured to receive via the user interface an indication of a desired relative position as determined in any of the methods and by any of the processing circuits as described herein. The data processor can be configured to compare this desired relative position with an actual relative position and provide feedback of the difference to the user via the user interface and the data processor may be configured to generate an information signal to be provided by the interface to the user when such desired position has been reached. Such information signal may be one for a visible tactile and/or audible indication by the user interface with appropriate devices such as speaker, tactile feedback device and/or display. One preferred example is to use a display device to display an indication.

For example, the user interface 990 may provide an indication to a user that the ultrasound transceiver 910 is inside the lumen of the interventional device 925. The user may then cause the ultrasound transceiver 910 to be moved further along the lumen in order to reach an opening of the interventional device 925. Once the ultrasound transceiver has been moved, the data processor 940 may determine a new relative position of the ultrasound transceiver based on the ultrasound echo corresponding to the new position.

In some embodiments, the system 900 may further comprise a retrofit device 950 that may be used to move the ultrasound transceiver 910 through the interventional device 925. The retrofit device 950 may be a guidewire or a catheter with a smaller diameter than the inner diameter of the lumen of the interventional device 925, or any other elongate interventional device suitable for moving the ultrasound transceiver 910 through the interventional device 925. The ultrasound transceiver 910 may be attached to one end of the retrofit device 950. For example, the ultrasound transceiver 910 may be attached to a tip of a guidewire or catheter.

In some embodiments, the retrofit device may comprise part of a wired connection between the ultrasound transceiver 910 and the transceiver controller 930.

In some embodiments, the system 900 may further comprise a clamp 960 for locking the ultrasound transceiver 910 in a fixed position with respect to an opening of the interventional device 925. The clamp 960 may, for example, be attached to the interventional device 925, for example to a Luer lock of the interventional device 925, and the ultrasound transceiver 910 and retrofit device 950 may be inserted into the lumen of the interventional device 925 through the clamp 960.

In response to the relative position not being the relative position desired by a user of the system 900, the clamp 960 may be disengaged to allow the ultrasound transceiver 910 to be moved for example to a desired relative position.

In response to the relative position being a desired relative position for example during further treatment of a subject using the interventional device, the clamp 960 may be engaged to lock the retrofit device 950, and therefore the ultrasound transceiver 910 attached to the retrofit device 950 to keep it in the desired relative position. A desired relative position, for example, can be such that the ultrasound transceiver is at or close to a distal opening at a tip of the interventional device 925, such as a needle opening or catheter tip or endoscope tip.

A desired relative position may be, for example, the position at which the one or more characteristics of the one or more echoes first display a difference from the one or more characteristics of the one or more echoes when the ultrasound transceiver 910 is known to be inside the interventional device 920, as the ultrasound transceiver moves from inside the interventional device towards an opening of the device.

In another example, the desired relative position may be the position just before the one or more characteristics of the one or more echoes first display a difference from the one or more characteristics of the one or more echoes when the ultrasound transceiver 910 is known to be inside the interventional device 920, as the ultrasound transceiver moves from inside the interventional device towards an opening of the device. A user of the system may place the ultrasound transceiver 910 in this position by moving the ultrasound transceiver 910 through the lumen of the interventional device 920 until a change in the one or more characteristics of the one or more echoes is detected, then retracting the ultrasound transceiver slightly.

In yet another example, the desired relative position may be determined to be in the middle of the position range over which the one or more characteristics of the one of more echoes changes as the ultrasound transceiver 910 moves from the inside of the interventional device 920 to outside the interventional device. This may, for example, be determined by moving the ultrasound transceiver 910 through the lumen of the interventional device 920 until a change in the one or more characteristics of the one or more echoes is detected, then continuing to move the ultrasound transceiver 910 through the opening of the interventional device 920 and away from the interventional device until it is detected that further moving the ultrasound sensing system 910 no longer results in a change in the one or more characteristics of the one or more echoes. The ultrasound transceiver system 910 may then be retracted back to the midpoint in the position range over which changes in the one or more characteristics of the one or more echoes were detected.

The system comprising the retrofit device 950, the interventional device 925 the controller 930 the processing circuit 940 and optionally the interface 990 may be used in conjunction with an US imaging apparatus as described in part with reference to FIG. 1. The system and apparatus may be separate entities, but alternatively and advantageously may also be integrated fully or in part. In such case the apparatus is considered to be integrated in the system 900.

Thus, in some embodiments, the system 900 may further comprise an ultrasound imaging probe 970, configured to transmit and receive ultrasound imaging beams and communicatively connected, via a wire or wirelessly, to an Ultrasound imaging controller 980. When systems are integrated, the controllers 930 and 980 may for example be integrated in one controller having both functionalities. For such an integrated controller there may be appropriate connection interfaces for connecting the retrofit device and the US imaging probe. There may be more than one retrofit device and/or more than one US imaging probes connectable to the respective controllers or integrated controller.

In embodiments, the ultrasound imaging probe 970 is an external US imaging probe, i.e. is configured for use outside of a subject. It is noted that other US imaging probes may be used such as for example internal imaging probes which can be used inside a subject. Examples of such internal probes are TEE or ICE US probes. These are well known in the art and will not be described here in detail. One important function of the US imaging probe of the system is that it can image a region of interest of a subject. The outside probe 970 will be used as example to describe how it may work together with the ensemble of retrofit device 950 and interventional device 925.

The system having the interventional device, the retrofit device and the US imaging probe and controller therefore may be used by a user to guide him while bringing a part of the interventional device to a desired location in the subject. The transceiver of the retrofit device therein may serve as a tracking tool that can be located by the US imaging system. Thus, existing interventional devices that are difficult to track with US imaging may then be retrofit to be at least temporarily (while they are together with a retrofit device) observable with US imaging. Furthermore, once such desired location has been reached, the retrofit device may be removed while keeping the interventional device at the desired location. Thus, the retrofitting may save cost of manufacture of interventional devices and/or may benefit use of US imaging during interventional procedures wherein existing interventional devices are used that are difficult to track with US imaging.

Thus in embodiments of the system, the US probe 970 and the US imaging controller 980 are capable of locating the position of the ultrasound transceiver 910 within a subject when it is inserted in a subject. This may be done for example when the transceiver is at or close to an opening of the interventional device 925, such as the tip of such device. The ultrasound imaging probe 970 may then be used to determine a location of the opening of the interventional device 925 within the subject, through determination of the location of the transceiver.

Tracking of an US transceiver or sensor by an US imaging system is known in the field. While more detailed description of such methods and device may be found elsewhere an exemplary system is described below.

In embodiments, the ultrasound imaging probe 970 may transmit an ultrasound imaging beam that is received by the ultrasound transceiver 910, and the system 900 is then further configured to determine a position of the ultrasound transceiver 910 relative to the ultrasound imaging probe 970 based on a characteristic of this US beam. In such case the transceiver 910 serves as an US tracking sensor for sensing of parts of the US beam. The characteristics may for example include one or more of: an angle and/or a time between transmission and reception of the US beam. The characteristic of the beam then may be used to determine the position of the ultrasound transceiver 910 in an imaging plane of the ultrasound imaging probe 970, and the time between transmission and reception, that is, the time of flight of the beam, may be used to determine a distance between the ultrasound imaging probe 970 and the ultrasound transceiver 910.

Alternatively, the ultrasound transceiver 910 may be configured as an US tracking transmitter and be capable of transmitting an ultrasound beam that is received by the ultrasound imaging probe 970. The system 900 may then be configured to determine a position of the ultrasound transceiver 910 relative to the ultrasound imaging probe 970 based on a characteristic of this US beam, for example, an angle, and/or a time between transmission and reception of the beam.

In some embodiments, a plurality of beams at different angles may be transmitted by the ultrasound imaging probe 970 and received by the ultrasound transceiver 910, or transmitted by the ultrasound transceiver 910 and received by the ultrasound imaging probe 970, and the system 900 may be configured to determine a position of the ultrasound transceiver 910 relative to the ultrasound imaging probe 970 based on a largest received amplitude beam of the plurality of beams. The largest received amplitude beam is the closest of the plurality of beams to a straight line connecting the ultrasound transceiver 910 and the ultrasound imaging probe 970 and may therefore provide a more accurate relative position of the ultrasound transceiver 910 than any other beam of the plurality of beams.

The system may determine which of the plurality of beams has the largest received amplitude. Methods for determining a largest received amplitude beam of a plurality of beams will be apparent to the skilled person.

In some embodiments, the system 900 may further comprise an image generation processor 980, connected to the ultrasound imaging probe 970, the transceiver controller 930 and the display device 940. In some embodiments, the transceiver controller 930 and the image generation processor 980 may comprise a single processor.

The image generation processor 980 may receive ultrasound imaging beams from the ultrasound imaging probe 970, wherein the ultrasound imaging beams correspond to echoes of ultrasound imaging beams transmitted by the ultrasound imaging probe 970 into a region of a subject. The region of the subject may include the ultrasound transceiver 910. The image generation processor 980 may generate at least one ultrasound image of the region of the subject from the received ultrasound imaging beams and the display device 940 may obtain the at least one ultrasound image from the image generation processor 980 and display the at least one ultrasound image.

The image generation processor 980 may also receive the determined position of the ultrasound transceiver 910 relative to the ultrasound imaging probe 970 from the transceiver controller 930 and provide an indication of the position of the ultrasound transceiver 910 in/on the at least one ultrasound image based on the determined position. The display device 940 may obtain the indication of the position of the ultrasound transceiver 910 in the at least one ultrasound image from the image generation processor 980 and display the indication.

In this way, a user of the system 900 may readily identify a position of the ultrasound transceiver 910, and therefore a position of an opening of the interventional device 920, such as a tip of a catheter or needle, within the displayed ultrasound image, and determine whether the interventional device 920 is in a correct position for carrying out a medical procedure.

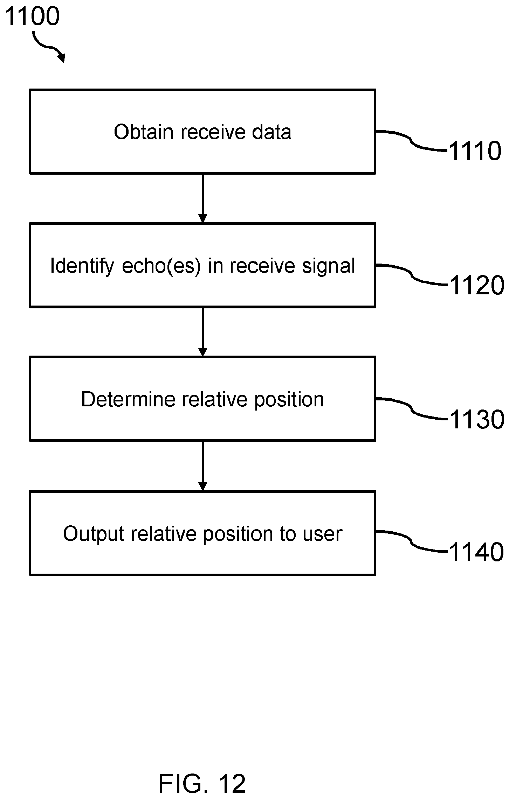

FIG. 12 illustrates a computer-implemented method 1200 for determining a relative position of an ultrasound transceiver with respect to an opening of an interventional device wherein the opening is in a lumen of the interventional device. Each step may represent a different action performed by a processor and may be performed by a respective module of the processing processor.

The method 1100 begins with step 1110, in which a receive signal is obtained from the ultrasound transceiver. The receive signal is responsive to echoes of ultrasound transmissions from the ultrasound sensor. The receive data thus may comprise echo information.

At step 1120, one or more echoes of an ultrasound transmission are identified in received data.

At step 1130, a relative position of the ultrasound transceiver is determined. The relative position is determined based on one or more characteristics of the identified one or more echoes as indicated herein before. For example, the one or more characteristics may comprise at least one of: an amplitude of the identified one or more echoes, an amplitude response or decay of the identified one or more echoes, and/or a time delay between an ultrasound transmission and the corresponding echo in the receive signal.

Step 1140 is optional and in this step the relative position or an indication of the relative position is output to a user.

It will be understood that the disclosed methods are computer-implemented methods. As such, there is also proposed a concept of a computer program product comprising computer program code means which, when executed on a computing device having a processing system, cause the processing system to perform all of the steps of any described method.

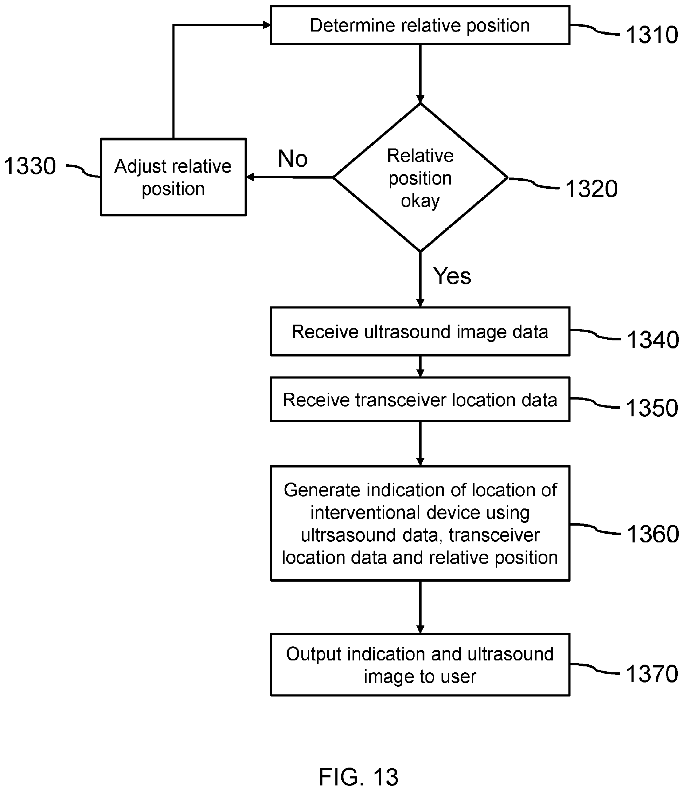

The method described may be repeated one or more times for example as part of a relative position calibration process. It may be used in a calibration process in which a relative position is calibrated against receive data or echo characteristics. For example, FIG. 13 shows step 1320 in which a relative position is checked. This may be done by a user after the relative position has been provided to the user. The user may respond an indicate yes when okay and no when not. If not, the user may readjust the relative position for example by manipulating the retrofit device with respect to the interventional device. The user may then cause the processor circuit to rerun step 1310 for another determination of the relative position as explained for example regarding FIG. 12 and description herein before. Such check may also be automatic. Thus steps 1320 and 1310 may be performed continuously while the user performs step 1330 to adjust the position. If the indication provided to the user is "okay" he may cause the continuous determination to stop and may fix the relative position as described herein before. Alternatively, or additionally, there may be a desired reference relative position. This may be user defined. The continuous determination my commence until, a user performed manipulation with step 1330, the reference value has been reached or passed. This process may be automatic as the processor may compare determined positions with reference positions or may compare characteristics of such positions with corresponding characteristics of the reference positions and a condition is reached automatically proceed to a next step, stop the procedure and/or output an indication of the final reference position.

The method may be used as part of a method for guiding navigation of an interventional device within a region of interest of a subject. Knowing the relative position as defined herein may be used to locate the opening of the interventional device (and therewith via the geometric properties of the interventional device also the location of at least part of the interventional device other than the opening) within the region of interest by tracking the transceiver with an ultrasound system having an ultrasound probe as described for example herein with reference to FIG. 1. In such case the transceiver is determined to have a relative position outside the opening but at a known distance therefrom. For example, it may be close to the opening or at the opening, but just outside. In that way it may be used as a beacon transmitting ultrasound that can be tracked using the ultrasound probe or be used as a passive sensor that can record signals transmitted by the ultrasound probe to localize it. References describing either techniques can be found elsewhere as provided herein before.

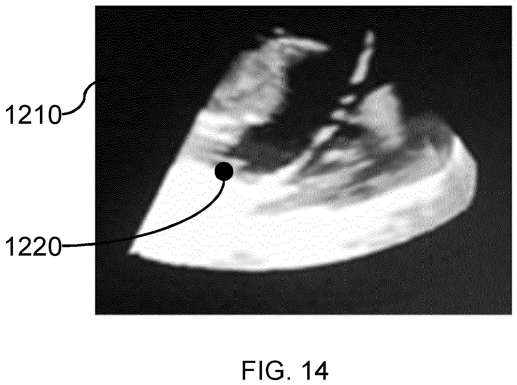

FIG. 14 illustrates a display of an ultrasound image 1210 and an indication 1220 of the position of the ultrasound transceiver 910 in the ultrasound image 1210, according to embodiments.

The display can be made with the user interface as described herein before. a display device 940.

The displayed ultrasound image 1210 and indication 1220 may be obtained with methods and systems and parts thereof as described herein above.

The indication 1220 comprises a marker superimposed on the ultrasound image 1210 at a position corresponding to the position of the ultrasound transceiver 910 in the ultrasound image 1010. The image and marker may be based on overlay of the marker on the image and the data can be generated by the data processor for display by a display of the user interface.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" and "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

Summarizing, there has been disclosed a system and a method for determining a relative position of an ultrasound transceiver with respect to an opening of a interventional device where the opening is within a lumen of the interventional device. Ultrasound transmissions from an ultrasound transceiver, capable of moving through at last part of the lumen and the opening are reflected off the wall or boundary of the lumen. The ultrasound transceiver may receive one or more echoes of the ultrasound transmissions and generate a receive signal comprising the one or more echoes and transmit receive data comprising one or more characteristics of the echo to a data processor. The data processor is configured to identify from the receive data the one or more characteristics and determine a relative position of the ultrasound transceiver with respect to the opening based on the one or more characteristics. A transceiver may be part of a retrofit device with which the interventional device may be equipped during an interventional procedure. After having determined the relative position, a location within a subject of the interventional device equipped with the retrofit device may be determined through tracking of the transceiver by an US imaging apparatus during the procedure.

## Claims

1. A device for determining a relative position of an ultrasound transceiver (110, 910) with respect to an opening (125) of an interventional device (120, 920), wherein the ultrasound transceiver is moveable with respect to the opening and is capable of providing ultrasound transmissions and of providing a receive signal comprising echoes of the ultrasound transmissions, the device comprising a processor circuit comprising:
- an input configured to receive data (915) comprising or representative of at least part of the receive signal;
- a data processor communicatively coupled to the input and configured to:
- identify in the receive data (915) the one or more characteristics; and
- determine, based on the identified one or more characteristics, an indication of the relative position; and
- optionally, an output adapted to provide result data comprising the indication to a user interface.

2. The device of claim 1, wherein the one or more characteristics comprise or consist of an amplitude of the one or more echoes and/or an amplitude response of the one or more echoes.

3. The device of claim 1 or 2, wherein the one or more characteristics comprise or consist of a non-zero time delay between an ultrasound transmission and the one or more echoes.

4. The device of any of claims 1 to 3, adapted to determine the relative position by comparing the one or more characteristics with one or more corresponding reference values.

5. The device of any of claims 1 to 4, wherein the data processor is adapted to identify a first echo and a second echo received after the first echo, and determine the relative position by comparing the one or more characteristics of the first echo with the corresponding one or more characteristics of the second echo.

6. The device of any of the previous claims, wherein the processing circuit has a further input for receiving ultrasound image data comprising an ultrasound image of a region of interest of a subject and location data representative of a transceiver location of the ultrasound transceiver within the ultrasound image, wherein the data processor is configured to generate the result data to comprise the ultrasound image and the transceiver location and/or the location of the opening of the interventional device.

7. A system (900) comprising:
- a device of any one of the claims 1 to 6 comprising the output, and
- a user interface communicatively coupled to the output and adapted to provide the indication to a user such as a caregiver, medical practitioner or subject.

8. A system (900) as claimed in claim 7, comprising the ultrasound transceiver and the interventional device (120, 920).

9. A system (900) of claim 8, wherein the ultrasound transceiver is moveable through the opening, the system comprising a retrofit device (950) adapted to move the ultrasound transceiver (110, 910) through the opening of the interventional device (120, 920).

10. A system (900) of any one of claims 7 to 9, further comprising a locking mechanism (960) for locking the ultrasound transceiver (110, 910) in a fixed position with respect to the opening (125).

11. A system (900) of any of claims 7 to 10, wherein the system comprises r consists of an ultrasound imaging system comprising an ultrasound imaging probe (970) adapted to transmit and receive ultrasound for generating an ultrasound image of a region of a subject and wherein the system or device is further configured to generate an indication of a location of the ultrasound transceiver in the ultrasound image based on an ultrasound based interaction of the ultrasound transceiver with the ultrasound imaging probe.

12. A computer implemented method for determining a relative position of an ultrasound transceiver (110, 910) with respect to an opening (125) of an interventional device (120, 920), wherein the ultrasound transceiver is moveable with respect to the opening and is capable of providing ultrasound transmissions and of providing a receive signal comprising echoes of the ultrasound transmissions, the method comprising using a processor circuit for:
- receiving or generating receive data (915) comprising or being representative of at least part of the receive signal;
- identifying in the receive data (915) the one or more characteristics; and
- determining, based on the identified one or more characteristics, an indication of the relative position; and
- optionally, providing result data comprising the indication to a user interface.

13. The method of claim 12, wherein the one or more characteristics comprise or consist of an amplitude of the one or more echoes and/or an amplitude response of the one or more echoes.

14. The method of any one of claim 12 or 13, wherein the one or more characteristics comprise or consist of a non-zero time-delay between an ultrasound transmission and the one or more echoes.

15. The method of any of claims 12 to 14, wherein the determining the relative position comprises comparing the one or more characteristics with one or more corresponding reference values.

16. The method of any of the claims 12 to 15, wherein identifying the one or more characteristics comprises identifying a first echo and a second echo received after the first echo, and determining the relative position comprises comparing the one or more characteristics of the first echo with the corresponding one or more characteristics of the second echo.

17. The method of any of the claims 12 to 16, comprising receiving or generating ultrasound image data comprising an ultrasound image of a region of interest of a subject and receiving or generating location data representative of a transceiver location of the ultrasound transceiver within the ultrasound image, and generating result data comprising the ultrasound image and the transceiver location and/or the location of the opening of the interventional device.

18. The method of any of the previous claims wherein the steps of identifying the one or more characteristics and determining the indication of the relative position and, optionally the transceiver location, are repeated at least once.

19. A computer program product comprising computer program code which, when executed by the processor circuit and/or data processor, causes the device or system to perform the steps of the method (1100) according to any one of claims 12 to 18.

20. A computer readable medium or carrier comprising a computer program product of claim 19.
